# EUROPÄISCHE PATENTANMELDUNG

(11) **EP 1 997 537 A2**
(43) Veröffentlichungstag der Anmeldung: **03.12.2008**
(21) Anmeldenummer: 08104065.1
(22) Anmeldetag: 22.05.2008
(51) Int. Cl.: A61Q 19/10, A61K 8/365, A61K 8/368, A61K 8/97, A61K 8/81, A61Q 19/00

(54) **Kosmetisches Hautreinigungsmittel**

(30) Priorität: 23.05.2007 DE 102007024138
(71) Anmelder: Coty Prestige Lancaster Group GmbH, 55116 Mainz (DE)
(72) Erfinder: Golz-Berner, Karin, 98000 Monaco (MC); Zastrow, Leonhard, 98000 Monaco (MC)
(74) Vertreter: Ziebig, Marlene

(57) **Zusammenfassung**

Die Erfindung betrifft ein kosmetisches Hautreinigungsmittel, das insbesondere auch gegen Akne vulgaris wirksam ist. Das Mittel hat einen pH-Wert im Bereich von 3,0 bis 4,5, wobei der Säurebildner aus der Gruppe ausgewählt ist, die wenigstens aus Milchsäure und Salicylsäure besteht, das Mittel ein Copolymer aus einem Acryloyltaurat und Vinylpyrrolidon umfaßt sowie einen Extrakt der Alge Laminaria saccharina. Das Mittel ist trotz des niedrigen pH-Wertes emulsionsstabil und zeigt eine gute, insbesondere langanhaltende Anti-Akne-Wirkung.

## Beschreibung

Die Erfindung betrifft ein kosmetisches Hautreinigungsmittel, das insbesondere auch gegen Akne vulgaris wirksam ist. Die Zusammensetzung ist trotz eines niedrigen pH-Wertes emulsionsstabil und zeigt eine lang anhaltende Anti-Akne-Wirkung.

Die US 4,355,028 offenbart ein topisches therapeutisches Mittel zur Behandlung von Akne vulgaris, das ein Gemisch von Salicylsäure und Benzoylperoxid enthält, wobei nur bei relativ hohen Konzentrationen von 5 % gute Ergebnisse erreicht werden, deren Nachhaltigkeit jedoch nicht dargestellt wurde.

Aus der US 5,876,737 ist ein Verfahren zur Behandlung dermatologischer Erkrankungen u.a. von Akne bekannt, bei dem als Wirkstoff Saligenin-β-glucosid (Salicin) verwendet wird.

Die EP 573 253 B1 beschreibt eine antibakterielle Zusammensetzung, die u.a. Haarverlust reduziert sowie Hautschäden durch Akne, und die ein spezielles C6-C12-Acyllactat als Wirkstoff enthält.

US 2006/0165741 A1 beschreibt eine stabile Hautpflegezusammensetzung mit niedrigem pH-Wert, die Dihydroacetic Acid (INCI-Name) enthält. Die Stabilität der Formulierung wird durch Verwendung mindestens eines Öls als Träger, in dem die Dehydroessigsäure verteilt ist, gewährleistet. Vorzugsweise ist das Öl PPG-15 Stearylether.

Der vorliegenden Erfindung liegt die Aufgabe zugrunde, ein Hautreinigungsmittel mit Anti-Akne-Wirkung bereitzustellen, das bei gleichzeitig hoher und langanhaltender Wirksamkeit die erforderliche Lager- und Anwendungsstabilität hat.

Die Aufgabe wird gelöst durch ein Hautreinigungsmittel mit einem pH-Wert im Bereich von 3,0 bis 4,5, wobei der Säurebildner wenigstens aus Milchsäure und Salicylsäure besteht, und das Mittel ein Copolymer aus einem Acryloyltaurat und Vinylpyrrolidon und einen Extrakt der Alge Laminaria saccharina sowie weiterhin kosmetisch übliche Hilfsstoffe, Trägerstoffe und Wirkstoffe umfasst.

Der Säurebildner besteht vorzugsweise wenigstens aus Milchsäure, Salicylsäure und Citronensäure, ganz besonders bevorzugt aus Milchsäure, Salicylsäure, Citronensäure und einem Citronensäuresalz, vorzugsweise dem Natriumsalz.

Als weiteren kosmetischen Wirkstoff umfasst das erfindungsgemäße Hautreinigungsmittel bevorzugt einen Pflanzenextrakt, ausgewählt aus der Gruppe, bestehend aus Ananasextrakt, Orangenextrakt, Zitronenextrakt, Bambusstengelextrakt, Hamamelis virginiana-Extrakt, Prunus armeniaca-Samenextrakt, Evernia furfuracea-Extrakt, Evernia prunastri-Extrakt, Peumus boldus-Extrakt, Angelica archangelica-Extrakt, Pongamia pinnata-Extrakt, Camellia sinensis-Extrakt, Coffea arabica-Extrakt und Gemischen davon.

Besonders bevorzugt kann als Pflanzenextrakt Ananas-, Orangen- oder Zitronenextrakt oder ein Gemisch daraus enthalten sein.

In einer anderen bevorzugten Asuführungsform kann der Pflanzenextrakt ausgewählt sein aus der Gruppe bestehend aus Bambusstengelextrakt, Hamamelis virginiana-Extrakt, Angelica archangelica-Extrakt, Pongamia pinnata-Extrakt, Camellia sinensis-Extrakt, Coffea arabica-Extrakt und Gemischen davon.

Das enthaltene Copolymer ist bevorzugt ein Ammonium Acryloyldimethyltaurat/VP Copolymer, vorzugsweise das unter der Handelsbezeichnung Aristoflex^{®} AVC erhältliche Copolymer der Clariant GmbH.

Es wurde gefunden, dass die Kombination von Salicylsäure, Milchsäure und einem Extrakt der Alge Laminaria saccharina eine besonders hohe Wirksamkeit und vor allem eine langanhaltende Wirksamkeit gegen Akne zeigt und auch gegen andere Hautunreinheiten sehr wirksam ist. Zwar ist die Wirksamkeit von Salicylsäure und von Phlorogine, dem wesentlichen Wirkstoff des Laminaria-Extraktes, für sich gegen Akne oder seborrheische Haut bekannt, jedoch ist insbesondere die Langzeitwirkung nicht immer zufriedenstellend.

Das erfindungsgemäße Mittel hat einen pH-Wert im Bereich von pH 3-4. Bei diesen niedrigen pH-Werten, die durch die für die Wirksamkeit notwendigen Säuren bedingt sind, ist die Stabilität von Emulsionen üblicherweise sehr schlecht, d.h. es kommt relativ schnell zu Phasentrennungen und starken Viskositätsverringerungen. Der Einsatz von vernetzten Siliconpolymeren führt dabei beispielsweise zu keinen Verbesserungen.

Es wurde weiterhin gefunden, dass die Einbeziehung eines Verdickungsmittels auf Basis eines Copolymeren von Vinylpyrrolidon und Acryloyltaurat zu stabilen Emulsionen mit Viskositäten im Bereich von 50 000 - 200 000 mPa·s auch bei niedrigen pH-Werten führt. Die Viskositätsmessung erfolgte dabei nach dem Brookfield-Verfahren mit den Spindeln TC/TD/TE bei 25°C im Bereich von 50-75 % der Spindelgeschwindigkeiten.

Der pH-Wert des erfindungsgemäßen Hautreinigungsmittels liegt bevorzugt im Bereich von 3,0 bis 4,0, speziell bei 3,3 - 3,9.

In einer bevorzugten Ausführungsform der Erfindung liegt der Anteil des Säurebildners im Hautreinigungsmittel bei 0,3 bis 1,8 Gew.%, bezogen auf das Gesamtgewicht des Mittels.

Der Anteil an Salicylsäure in dem erfindungsgemäßen kosmetischen Hautreinigungsmittel liegt vorzugsweise im Bereich von 0,2 bis 0,8, Gew.%, besonders bevorzugt im Bereich von 0,2 - 0,45 Gew-%, bezogen auf das Gesamtgewicht des Mittels.

Der Anteil an Milchsäure liegt vorzugsweise im Bereich von 0,1 bis 1,0, Gew.%, besonders bevorzugt bei 0,2 - 0,8 Gew-%.

Der Anteil des Laminaria saccharina-Extraktes in dem erfindungsgemäßen kosmetischen Hautreinigungsmittel liegt vorzugsweise im Bereich von 0,4 bis 1,3 Gew.%, besonders bevorzugt im Bereich von 0,5 - 1,0 Gew-%, bezogen auf das Gesamtgewicht des Mittels.

Der Anteil des Acryloyltaurat/Vinylpyrolidon-Copolymers liegt vorzugsweise im Bereich von 0,5 bis 2,9 Gew.%, besonders bevorzugt von 0,5 - 0,8 Gew-%, bezogen auf das Gesamtgewicht des Mittels.

Das erfindungsgemäße Mittel kann weiterhin kosmetische Hilfs- und Trägerstoffe umfassen, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und/oder unpolare Öle, Polymere, Copolymere, Emulgatoren, Wachse und/oder Stabilisatoren.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Als Ester oder Ether sind zum Beispiel geeignet Glyceryl Stearate, Ethylhexyl Palmitate, Propylene Glycol Dioctanoate, Propylene Glycol Dicaprylate-2,30-Dicaprate, Tridecyl Stearate/ Neopentyl Glycol Dicaprylate Dicaprate/Tridecyl Trimellitate, Neopentyl Glycol Dioctanoate, Isopropyl Myristate, Diisopropyl Dimer Dilinoleate, Trimethylpropane Triisostearate, Myristyl Ether, Stearyl Ether, Cetearyl Octanoate, Butyl Ether, Dicaprylyl Ether, PPG1-PEG9 Lauroyl Glycol Ether, PPG15 Stearyl Ether, PPG14 Butyl Ether.

Weitere Verdickungsmittel können sein Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Als Hilfs- oder Trägerstoffe eingesetzte Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titandioxid, Glimmer, Kaolin, manganhaltige Tone, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen und gemahlene Pflanzenteile.

Das erfindungsgemäße Hautreinigungsmittel kann als weitere Wirkstoffe z. B. anorganische und organische Lichtschutzmittel, Selbstbräunungsmittel, Radikalfänger, Feuchthaltemittel, Vitamine, Enzyme, pflanzliche Wirkstoffe, Polymere, Melanin, Antioxidationsmittel, entzündungswidrige natürliche Wirkstoffe und mit Sauerstoff beladene asymmetrische lamellare Aggregate gemäß WO 94/00109 enthalten.

Bevorzugte Wirkstoffe, die den Zustand der Haut positiv beeinflussen, sind insbesondere solche zur positiven Beeinflussung der Altershaut, insbesondere in Kombination mit Biochinonen, insbesondere Ubichinon Q10, Kreatin, Kreatinin, Camitin, Biotin, Isoflavone, Cardiolipin, Liponsäure, Anti Freezing Proteine, Arctiin, Hopfen- und Hopfen-Malz-Extrakte.

Fördernde Mittel zur Restrukturierung des Bindegewebes sind insbesondere Isoflavonoide.

Wirkstoffe zur Unterstützung der Hautfunktionen bei trockener Haut sind insbesondere Vitamin C, Biotin, Camitin, Kreatin, Propionsäure, Grüntee-Extrakte, Eucalyptusöl, Harnstoff und Mineralsalze, insbesondere NaCl, Meeresmineralien sowie Osmolyte.

Wirkstoffe zur Linderung und/oder positiven Beeinflussung von irritativen Hautzuständen sind insbesondere Sericoside, verschiedene Extrakte des Süßholzes, Licochalcone, insbesondere Licochalcone A, Silymarin, Silyphos, Dexpanthenol.

Modulatoren der Pigmentierung sind insbesondere Tyrosinsulfat, 8-Hexadecen-1,16-dicarbonsäure, Liponsäure, Liponamid, verschiedene Extrakte des Süßholzes, Kojisäure, Hydrochinon, Arbutin, Fruchtsäuren, insbesondere α-Hydroxy-Säuren (AHAs), Uvae ursi, Ursolsäure, Ascorbinsäure, Grüntee-Extrakte, Aminoguanidin, Pyridoxamin.

Wirkstoffe, die eine verstärkte oder schnellere Bräunung der Haut herbeiführen, sind insbesondere Lipofuscine, Nukleinsäure, Oligonukleotide, Purine, Pyrimidine, Dihydroxyaceton, Erythrulose, NO-freisetzende Substanzen. Weitere Wirkstoffe können sein Phytoen, Phytofluen, ζ-Carotin, Neurosporin, Lycopin, β-Carotin, Squalen, Variabilin, Phytansäure und/oder Phytol.

Es kann weiterhin vorteilhaft sein, den erfindungsgemäßen Hautreinigungsmitteln entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester; Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Die Erfindung betrifft auch die Verwendung des kosmetischen Mittels zur Anti-Akne-Behandlung. Die Behandlung erfolgt dabei vorzugsweise über einen Zeitraum von 4 bis 8 Wochen.

Das erfindungsgemäße kosmetische Hautreinigungsmittel kann z.B. in Cremes, Masken, Körperlotionen, Reinigungsmilch, Make up's, Lippenstiften, Lippenbalsam, Körperpuder, Augenkosmetik, Haarmasken, Haarspülungen, Haarshampoos, Duschgelen, Duschölen, Badeölen, Rollern oder als Serum sowie in Sonnenschutzzubereitungen und Hautbräunungsmittel aller Art verwendet werden. Die Herstellung derartiger Produkte erfolgt auf übliche Weise, wie sie dem Fachmann auf diesem Gebiet bekannt ist.

Gegenstand der vorliegenden Erfindung ist auch die vorstehend beschriebene Zusammensetzung zur Behandlung von Akne.

Die Erfindung soll nachstehend durch Beispiele näher erläutert werden. Alle Angaben erfolgen in Gewichtsprozent (Gew.%), sofern nichts anderes angegeben ist.

### Beispiel 1/1a Anti-Akne Reiniger

| | Beispiel 1 | Beispiel 1a |
|---|---|---|
| Wasser | q.s. ad 100 | q.s. ad 100 |
| Propylenglycol | 0,1 - 0,5 | 0,2 |
| Dinatrium EDTA | 0,1 - 0,2 | 0,1 |
| Natriumchlorid | 0,6 - 1,0 | 0,7 |
| Milchsäure | 0,5 - 0,8 | 0,6 |
| Parfüm | 0,4 - 0,6 | 0,4 |
| Ammonium Acryloyldimethyl- | | |
| taurate / VP Copolymer | | |
| (Aristoflex® AVC) | 2,0 - 2,7 | 2,4 |
| Sodium Laureth Sulfate | | |
| (Tensid) | 8,0 - 10,0 | 9,5 |
| Salicylsäure | 0,5 - 0,6 | 0,5 |
| Bambusstengelextrakt | 3,0 - 5,0 | 3,5 |
| Hamamelisextrakt | 1,0 - 5,0 | 1,5 |
| Konservierungsmittel | 0,5 - 1,0 | 0,6 |
| Laminaria saccharina Extract | 0,5 - 1,0 | 0,7 |
| Prunus armeniaca Seed Powder | | |
| & Evernia furfuracea Extract | | |
| & Evernia prunastri Extract | 0,8 - 1,2 | 1,0 |
| Cocamidopropyl Betaine | | |
| (Tensid) | 8,0 - 10,0 | 10,0 |
| Disodium Laureth Sulfosuccinate | | |
| & Sodium Lauryl Sulfoacetate | | |
| (Tensid) | 8,0 - 10,0 | 9,0 |
| Propylene Glycol & PEG-55 | | |
| Propylene Glycol Oleate | 4,5 - 6,0 | 4,8 |

Zu Wasser wurden die Tenside zugegeben, danach unter Rühren das auf etwa 40°C erwärmte Ammonium Acryloyldimethyltaurate/VP Copolymer und bei etwa 20-25°C die anderen Rohstoffe langsam unter Rühren. Der pH-Wert des Gemisches beträgt 3,8. Man erhält ein stabiles Produkt.

### Beispiel 2/2a Anti-Akne Roller

| | Beispiel 2 | Beispiel 2a |
|---|---|---|
| Wasser | q.s. ad 100 | q.s. ad 100 |
| Milchsäure | 0, 5 - 0,8 | 0, 65 |
| Laminaria saccharina Extract | 0,5 - 1,2 | 0, 6 |
| Ammonium Acryloyldimethyl- | | |
| taurate / VP Copolymer | | |
| (Aristoflex® AVC) | 0,5 - 1,0 | 0,5 |
| Ethanol | 20, 0 - 25,0 | 23,0 |
| Salicylsäure | 0, 4 - 0,6 | 0,45 |
| Citronensäure | 0,1 - 0,2 | 0, 1 |
| Natriumcitrat | 0,1 - 0,2 | 0,2 |
| Hamamelis Extract | 1, 0 - 2,0 | 1,2 |
| Peumus boldus Blattextrakt | 0, 7 - 1,5 | 0, 8 |

pH-Wert 3,6. Das Produkt ist lagerstabil.

### Beispiel 3/3a Anti-Akne Serum

| | Beispiel 3 | Phase | Beispiel 3a |
|---|---|---|---|
| PEG-100 Stearate & | | | |
| Glyceryl Stearate | 3,0 - 4,2 | A | 3,5 |
| Ethylhexyl Palmitate | 1,2 - 1,9 | A | 1,5 |
| Wasser | q.s. ad 100 | B | q.s. ad 100 |
| Salicylsäure | 0,2 - 0,5 | B | 0,3 |
| Trinatrium EDTA | 0,05 - 0,08 | A | 0,06 |
| Glycerin | 2,0 - 4,0 | A | 2,4 |
| Milchsäure | 0,1 - 0,5 | A | 0,2 |
| Xanthan Gum | 0,1 - 0,2 | A | 0,1 |
| Ammonium Acryloyldimethyl- | | | |
| taurate / VP Copolymer | | | |
| (Aristoflex^{®} AVC) | 0,6 - 2,0 | C | 0,8 |
| Siliconöl (1000 cSt) | 0,5 - 1,0 | A | 0,6 |
| Siliconöl (Cyclopentasiloxane | | | |
| & Cyclohexasiloxane | | | |
| & Cyclotetrasiloxane | 6,0 - 8,0 | A | 6,5 |
| Vinyl Dimethicone / | | | |
| Methicone Silsequioxane | 0,5 - 1,5 | A | 1,3 |
| Modifizierte Maisstärke | 5,5 - 7,0 | A | 5,5 |
| Zinkoxid | 0,01 - 0,02 | A | 0,01 |
| Nylon-12 | 0,5 - 1,0 | A | 0,6 |
| Kaolin | 0,5 - 1,0 | A | 0,7 |
| RPF-Komplex* | 0,5 - 1,0 | C | 0,5 |
| Retinylpalmitat & Tocopherol | 0,1 - 0,2 | A | 0,1 |
| Laminaria saccharina Extract | 0,5 - 1,0 | C | 0,6 |
| Hamamelisextract | 1,0 - 2,0 | C | 1,2 |
| Natriumhyaluronat-Liposome | 0,1 - 0,2 | C | 0,2 |
| Ethanol | 4,5 - 5,0 | C | 4,6 |
| Parfüm | 0,15 - 0,2 | C | 0,15 |
| Konservierungsmittel | 0,5 - 0,8 | C | 0,5 |

| | | | |
|---|---|---|---|
| *RPF-Komplex: Angelica archangelica-Wurzelextrakt, Pongamia pinnata-Samenextrakt, Camelia sinensis-Blattextrakt, Coffea arabica-Samenextrakt, Ethanol im Verhältnis 0,2 : 0,2 : 0,2 : 0,2 : 99,2 gemäß WO 2004/105704. | | | |

Die Bestandteile der Phase A werden auf ca. 60°C erwärmt, und die separat hergestellte Phase B wird ebenfalls auf diese Temperatur erwärmt und in die Phase A unter Rühren gegeben. Nach dem Abkühlen des Phasengemisches auf ca. 40°C wird die Phase C hinzugegeben und weitergerührt. Danach wird bei ca. 35°C unter Rühren die Phase D hinzugegeben. Der pH-Wert des Produktes beträgt 3,9. Das Serum ist emulsionsstabil.

### Beispiel 4/4a Anti-Akne Tagescreme mit Sonnenschutz SPF15

| Phase A | Beispiel 4 | Beispiel 4a |
|---|---|---|
| Wasser | q.s. ad 100 | q.s. ad 100 |
| Chlorphensin | 0,3 - 0,4 | 0,3 |
| Salicylsäure | 0,2 - 0,5 | 0,2 |
| Tetranatrium EDTA | 0,1 - 0,15 | 0,1 |
| Milchsäure | 0,5 - 0,6 | 0,5 |
| Glycerin | 3,0 - 5,0 | 3,5 |
| Retinylpalmitat & Tocopherol | 0,1 - 0,2 | 0,1 |

| Phase B | | |
|---|---|---|
| Ethylhexyl Methoxycinnamate BHT | 4,0 - 5,0 | 5,0 |
| Ethylhexyl Salicylate | 4,0 - 5,0 | 4,8 |
| Octocrylene | 1,0 - 2,0 | 1,2 |
| Butyl Methoxydibenzoylmethane | 2,0 - 2,5 | 2,0 |
| Benzophenone-3 | 2,0 - 2,5 | 2,2 |
| Glyceryl Stearate SE | 3,5 - 4,0 | 3,6 |
| Mikrokristallines Wachs | 1,0 - 2,0 | 1,1 |
| Cetylalkohol | 2,5 - 3,5 | 2,7 |
| PEG-40 Stearate | 2,5 - 3,0 | 2,5 |
| Stearinsäure & Palmitinsäure | 0,8 - 1,0 | 0, 8 |
| Tocopherol | 1,0 - 2,0 | 1,3 |
| Cyclopentasiloxane | | |
| & Cyclotetrasiloxane | 2,0 - 3,0 | 2,3 |
| Talkum | 2,5 - 3,0 | 2,5 |
| Mod. Maisstärke | 2,0 - 5,0 | 2,5 |
| Zinkoxid | 0,01 - 0,02 | 0,01 |

| Phase C | | |
|---|---|---|
| Ammonium Acryloyldimethyl- | | |
| taurate / VP Copolymer | | |
| (Aristoflex^{®} AVC) | 0,5 - 1,5 | 0,7 |
| Polyacrylamide & C13-14 | | |
| Isoparaffin & Laureth-7 | 1,0 - 1,5 | 1,1 |

| Phase D | | |
|---|---|---|
| Laminaria saccharina Extract | 0,5 - 1,0 | 0,6 |
| RPF-Komplex* | 0,5 - 1,0 | 0,5 |
| Konservierungsmittel | 0,5 - 1,0 | 1,0 |
| Natriumhyaluronat-Liposome | 0,2 - 0,5 | 0,3 |
| Hamamelisextrakt | 1,0 - 2,0 | 1,2 |
| Parfüm | 0,1 - 0,2 | 0,1 |
| Ethanol | 4,5 - 6,5 | 5,0 |

Die Herstellung erfolgt wie im Beispiel 3/3a. Der pH-Wert Beträgt 3,6. Die Emulsion ist sehr stabil.

### Beispiel 5 Vergleichsbeispiel

### Vergleich Anti-Akne-Mittel

Eine Gruppe von 15 Personen (9 männlich, 6 weiblich) mit einer mittelstarken Akne vulgaris und Komedonen unterschiedlicher Reife sowie Pusteln wurden auf unterschiedlichen Hautflächen gleichzeitig mit einem Serum gemäß Beispiel 3a (Produkt I) und einem Serum gemäß Beispiel 3a ohne Milchsäure und Salicylsäure (Produkt II) behandelt. Die Behandlung erfolgte einmal täglich über einen Zeitraum von 6 Wochen, woran sich ein weiterer Beobachtungszeitraum ohne Behandlung von 4 Wochen anschloss.

Die Wirkung der beiden Produkte wurde nach folgendem Schema bewertet:
1 schwache Wirkung = Verbesserung weniger als 25 %
2 mittlere Wirkung = Verbesserung >25 bis 50 %
3 gute Wirkung = Verbesserung >50 bis 75 %
4 sehr gute Wirkung = Verbesserung >75 bis 95 %.

Die Bewertung nach den Punktzahlen 1-4 erfolgte nach 10, 20, 30, 42 Tagen und im Beobachtungszeitraum nach insgesamt 55 und 70 Tagen. Es wurden die Mittelwerte aller Versuchsteilnehmer in der folgenden Tabelle eingesetzt.

| Tag | Produkt I | Produkt II |
|---|---|---|
| 0 | 0 | 0 |
| 10 | 1,5 | 1,1 |
| 20 | 2,3 | 1, 6 |
| 30 | 2, 6 | 1, 9 |
| 42 | 3,2 | 2,1 |
| 55 | 3,2 | 1,9 |
| 70 | 2,8 | 1,4 |

Aus den Ergebnissen ist klar ersichtlich, dass das erfindungsgemäße Hautreinigungsmittel (Produkt I) nach 6 Wochen Anwendung eine mehr als gute Wirkung erzielte im Gegensatz zu einer nur knapp mittleren Wirkung des Produktes II, und dass diese gute Wirkung ohne Weiterbehandlung auch nach 4 Wochen nahezu gut war. Dagegen fiel das Produkt II deutlich auf schwache Werte in diesem Zeitraum zurück.

## Patentansprüche

1. Kosmetisches Hautreinigungsmittel mit Anti-Akne-Wirksamkeit, **dadurch gekennzeichnet, dass** das Mittel einen pH-Wert im Bereich von 3,0 bis 4,5 hat, wobei der Säurebildner wenigstens aus Milchsäure und Salicylsäure besteht, das Mittel ein Copolymer aus einem Acryloyltaurat und Vinylpyrrolidon enthält sowie einen Extrakt der Alge Laminaria saccharina,
und das Mittel weiterhin kosmetisch übliche Hilfsstoffe, Trägerstoffe und Wirkstoffe enthält.

2. Hautreinigungsmittel nach Anspruch 1,
**dadurch gekennzeichnet, dass** der Säurebildner wenigstens aus Milchsäure, Salicylsäure und Citronensäure besteht, vorzugsweise aus Milchsäure, Salicylsäure, Citronensäure und einem Citronensäuresalz.

3. Hautreinigungsmittel nach Anspruch 1 oder 2,
**dadurch gekennzeichnet, dass** das Mittel als weiteren kosmetischen Wirkstoff einen Pflanzenextrakt enthält, ausgewählt aus der Gruppe, bestehend aus Ananasextrakt, Orangenextrakt, Zitronenextrakt, Bambusstengelextrakt, Hamamelis virginiana-Extrakt, Prunus armeniaca-Samenextrakt, Evernia furfuracea-Extrakt, Evernia prunastri-Extrakt, Peumus boldus-Extrakt, Angelica archangelica-Extrakt, Pongamia pinnata-Extrakt, Camellia sinensis-Extrakt, Coffea arabica-Extrakt und Gemischen davon.

4. Hautreinigungsmittel nach Anspruch 3, **dadurch gekennzeichnet, dass** der Pflanzenextrakt Ananas-, Orangen- oder Zitronenextrakt oder ein Gemisch davon ist.

5. Hautreinigungsmittel nach Anspruch 3,
**dadurch gekennzeichnet, dass** der Pflanzenextrakt ausgewählt ist aus der Gruppe bestehend aus Bambusstengelextrakt, Hamamelis virginiana-Extrakt, Angelica archangelica-Extrakt, Pongamia pinnata-Extrakt, Camellia sinensis-Extrakt, Coffea arabica-Extrakt und Gemischen davon.

6. Hautreinigungsmittel nach einem der Ansprüche 1 bis 5, **dadurch gekennzeichnet, dass** das Copolymer ein Ammonium Acryloyldimethyltaurat / VP Copolymer ist.

7. Hautreinigungsmittel nach einem der Ansprüche 1 bis 6, **dadurch gekennzeichnet, dass** der pH-Wert im Bereich von 3,0 bis 4,0 liegt, vorzugsweise 3,3 - 3,9.

8. Hautreinigungsmittel nach einem der Ansprüche 1 bis 7, **dadurch gekennzeichnet, dass** der Säurebildner insgesamt mit einem Anteil von 0,3 bis 1,8 Gew.% im Hautreinigungsmittel enthalten ist, bezogen auf das Gesamtgewicht des Hautreinigungsmittels.

9. Hautreinigungsmittel nach einem der Ansprüche 1 bis 8, **dadurch gekennzeichnet, dass** der Anteil des Laminaria saccharina-Extraktes im Hautreinigungsmittel 0,4 bis 1,3 Gew.% beträgt, vorzugsweise 0,5 bis 1,0 Gew.%, bezogen auf das Gesamtgewicht des Hautreinigungsmittels.

10. Hautreinigungsmittel nach einem der Ansprüche 1 bis 9, **dadurch gekennzeichnet, dass** der Anteil des Copolymers im Bereich von 0,5 bis 2,9 Gew.% liegt, vorzugsweise 0,5 - 0,8 Gew-%, bezogen auf das Gesamtgewicht des Hautreinigungsmittels.

11. Verwendung eines kosmetischen Hautreinigungsmittels gemäß den Ansprüchen 1 bis 10 zur Anti-Akne-Behandlung.

12. Zusammensetzung nach einem der Ansprüche 1 bis 10 zur Behandlung von Akne.
